# EUROPÄISCHE PATENTANMELDUNG

(11) **EP 4 043 429 A1**
(43) Veröffentlichungstag der Anmeldung: **17.08.2022**
(21) Anmeldenummer: 21156723.5
(22) Anmeldetag: 12.02.2021
(51) Int. Cl.: C07C 67/36, C07C 69/608

(54) **VERFAHREN ZUR ALKOXYCARBONYLIERUNG VON ALKYLHALOGENIDEN MITTELS KUPFER-KATALYSATORS**

(71) Anmelder: Evonik Operations GmbH, 45128 Essen (DE)
(72) Erfinder: WU, Xiao-Feng, 18059 Rostock (DE); GENG, Hui-Qing, 18059 Rostock (DE); BÖRNER, Armin, 18059 Rostock (DE); FRANKE, Robert, 45772 Marl (DE)
(74) Vertreter: Evonik Patent Association

(57) **Zusammenfassung**

Verfahren zur Alkoxycarbonylierung von Alkylhalogeniden mittels Kupfer-Katalysators.

## Beschreibung

Die Erfindung betrifft ein Verfahren zur Alkoxycarbonylierung von Alkylhalogeniden mittels Kupfer-Katalysators.

Die technische Aufgabe, welche der vorliegenden Erfindung zugrunde lag, ist die Bereitstellung eines Verfahrens, mit welchen Alkylhalogeniden alkoxycarbonyliert werden können. Mit diesem Verfahren soll eine gute Ausbeute erzielt werden.

Diese Aufgabe wird gelöst durch ein Verfahren nach Anspruch 1.

Verfahren umfassend die Verfahrensschritte:
a) Vorlegen eines Alkylhalogenids;
b) Zugabe eines Liganden gemäß Formel (I): 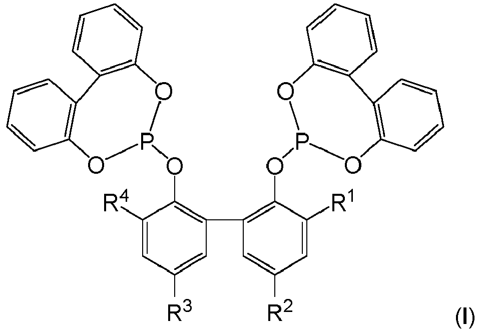 wobei
   R¹, R², R³, R⁴ jeweils unabhängig voneinander ausgewählt sind aus: -H, -(C₁-C₁₂)-Alkyl, -O-(C₁-C₁₂)-Alkyl;
c) Zugabe einer Verbindung, welche Cu umfasst;
d) Zugabe eines Alkoholats,
f) Zuführen von CO und H₂;
g) Erwärmen des Reaktionsgemisches aus a) bis f), wobei das Alkylhalogenid zu einem Ester umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis d) vorgelegt wurden. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Der Begriff -(C₁-C₁₂)-Alkyl bzw. -O-(C₁-C₁₂)-Alkyl umfasst geradkettige und verzweigte Alkylgruppen mit 1 bis 12 Kohlenstoffatomen. Vorzugsweise handelt es sich dabei um -(C₁-C₈)-Alkylgruppen bzw. -O-(C₁-C₈)-Alkylgruppen, besonders bevorzugt um -(C₁-C₄)-Alkylgruppen bzw. -O-(C₁-C₄)-Alkylgruppen.

In einer Variante des Verfahrens stehen R¹ und R⁴ für -(C₁-C₁₂)-Alkyl.

In einer Variante des Verfahrens stehen R¹ und R⁴ für -*^{ter}*Bu.

In einer Variante des Verfahrens stehen R² und R³ für -O-(C₁-C₁₂)-Alkyl.

In einer Variante des Verfahrens stehen R² und R³ für -OCH₃.

In einer Variante des Verfahrens weist der Ligand die Struktur (1) auf:

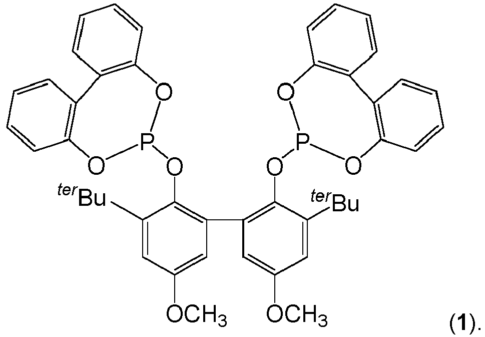

In einer Variante des Verfahrens handelt es sich bei dem Alkylhalogenid in Verfahrensschritt a) um ein lodid.

In einer Variante des Verfahrens ist die Verbindung, welche Cu umfasst, ausgewählt aus: CuCl₂, [Cu(OTf)], Cul, CuBr, Cu(OAc)₂.

In einer Variante des Verfahrens handelt es sich bei dem Alkoholat aus Verfahrensschritt d) um ein Natrium-Alkoholat oder Kalium-Alkoholat.

In einer Variante des Verfahrens handelt es sich bei dem Alkoholat aus Verfahrensschritt d) um ein Natrium-Alkoholat.

In einer Variante des Verfahrens erfolgt das Zuführen von CO in Verfahrensschritt f) mit einem Druck in dem Bereich von 1 bis 6 MPa (10 bis 60 bar).

In einer Variante des Verfahrens erfolgt das Erwärmen des Reaktionsgemisches in Verfahrensschritt g) auf eine Temperatur in dem Bereich von 80 °C bis 160 °C.

In einer Variante des Verfahrens umfasst dieses den zusätzlichen Verfahrensschritt f'): f') Zugabe eines Lösungsmittels.

In einer Variante des Verfahrens ist das Lösungsmittel ausgewählt aus: CH₃CN, DMSO, DMF, *^{tert}*BuOH, Toluol, Cyclohexan.

Im Folgenden soll die Erfindung anhand eines Ausführungsbeispiels näher erläutert werden.

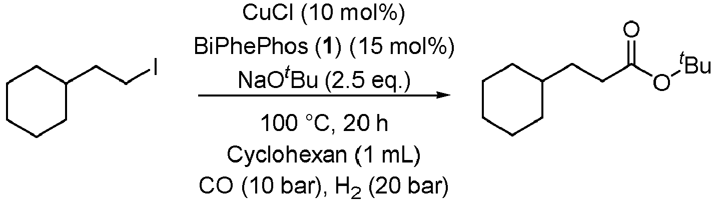

### BiPhePhos (1):

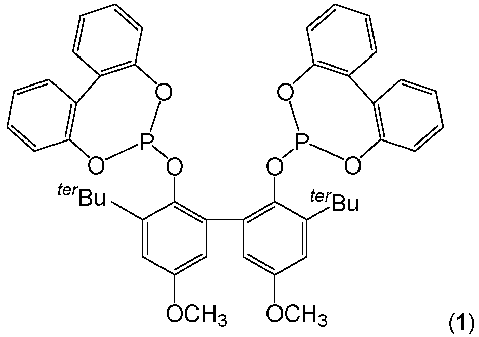

Ein Glasvial (4 mL) wurde mit CuCI (0,01 mmol, 1,0 mg, 0,1 eq), BiPhePhos (1) (0,015 mmol, 14,2 mg, 0,15 eq), (2-Jodethyl)cyclohexan 1 (0,1 mmol, 23,8 mg, 1,0 eq), NaOtBu (0,25 mmol, 48 mg, 2,5 eq) und einem Rührstab gefüllt. Das Vial wurde mit einem PTFE/Weißgummi-Septum (Wheaton 13 mm Septa) und einer Kunststoffkappe verschlossen und mit einer Nadel an die Gaszufuhr angeschlossen. Das Fläschchen wurde unter Vakuum evakuiert und dreimal mit Argon nachgefüllt. Dann wurde Cyclohexan (1,0 mL) mit einer Spritze unter Argon eingespritzt. Die Küvette (oder mehrere Küvetten) wurde in eine Platte gestellt, die in einen 300 mL Autoklaven der Serie 4560 von Parr Instruments überführt wurde. Nach dreimaligem Spülen des Autoklaven mit CO wurde ein Druck von 10 bar CO und 20 bar H₂ bei Raumtemperatur eingestellt. Dann wurde die Reaktion für 20 h bei 100 °C durchgeführt. Nach Beendigung der Reaktion wurde der Autoklav mit Eiswasser auf Raumtemperatur abgekühlt und der Druck wurde vorsichtig entspannt. Der Rückstand wurde durch Säulenchromatographie (Pentan/EA = 50/1) gereinigt, um das Produkt als farbloses Öl zu erhalten (15,7 mg, 74 % Ausbeute).

Der Versuch wurde unter analogen Bedingungen mit den Vergleichsliganden P(OPh)₃ (2) und P(OEt)₃ (3) wiederholt.

### Reaktionsbedingungen:

T = 100 °C, p(CO) = 10 bar, p(H₂) = 20 bar, t = 20 h, Lösungsmittel = Cyclohexan.

Die Versuchsergebnisse sind in der nachfolgenden Tabelle aufgeführt:

| Ligand | Ausbeute [%] |
|---|---|
| (**1**)* | 74 |
| (**2**) | 71 |
| (**3**) | 23 |

| | |
|---|---|
| * erfindungsgemäßes Verfahren | |

Mit dem erfindungsgemäßen Verfahren konnte eine bessere Ausbeute erzielt werden als in den beiden Vergleichsversuchen.

Wie das Ausführungsbeispiel zeigt, wird die Aufgabe durch das erfindungsgemäße Verfahren gelöst.

## Patentansprüche

1. Verfahren umfassend die Verfahrensschritte:
a) Vorlegen eines Alkylhalogenids;
b) Zugabe eines Liganden gemäß Formel (I): 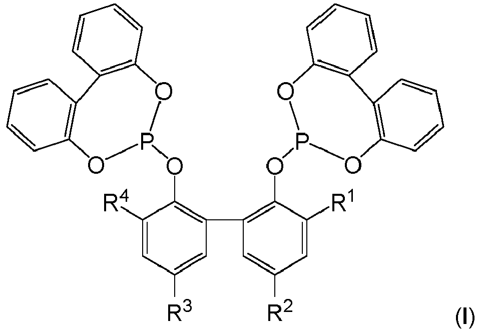 wobei
R¹, R², R³, R⁴ jeweils unabhängig voneinander ausgewählt sind aus: -H, -(C₁-C₁₂)-Alkyl, -O-(C₁-C₁₂)-Alkyl;
c) Zugabe einer Verbindung, welche Cu umfasst;
d) Zugabe eines Alkoholats,
f) Zuführen von CO und H₂;
g) Erwärmen des Reaktionsgemisches aus a) bis f), wobei das Alkylhalogenid zu einem Ester umgesetzt wird.

2. Verfahren nach Anspruch 1,
wobei R¹ und R⁴ für -(C₁-C₁₂)-Alkyl stehen.

3. Verfahren nach einem der Ansprüche 1 oder 2,
wobei R¹ und R⁴ für -*^{ter}*Bu stehen.

4. Verfahren nach einem der Ansprüche 1 bis 3,
wobei R² und R³ für -O-(C₁-C₁₂)-Alkyl stehen.

5. Verfahren nach einem der Ansprüche 1 bis 4,
wobei R² und R³ für -OCH₃ stehen.

6. Verfahren nach einem der Ansprüche 1 bis 5,
wobei der Ligand die Struktur (1) aufweist:

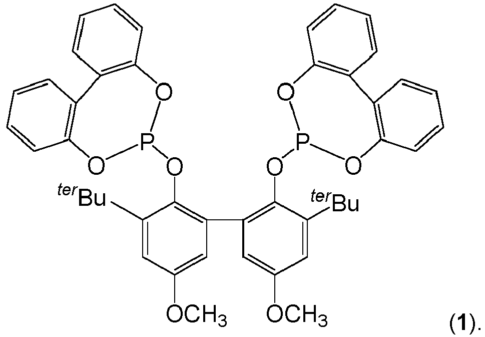

7. Verfahren nach einem der Ansprüche 1 bis 6,
wobei es sich bei dem Alkylhalogenid in Verfahrensschritt a) um ein lodid handelt.

8. Verfahren nach einem der Ansprüche 1 bis 7,
wobei die Verbindung, welche Cu umfasst, ausgewählt ist aus: CuCl₂, [Cu(OTf)], Cul, CuBr, Cu(OAc)₂.

9. Verfahren nach einem der Ansprüche 1 bis 8,
wobei es sich bei dem Alkoholat aus Verfahrensschritt d) um ein Natrium-Alkoholat oder Kalium-Alkoholat handelt.

10. Verfahren nach einem der Ansprüche 1 bis 9,
wobei das Zuführen von CO in Verfahrensschritt f) mit einem Druck in dem Bereich von 1 bis 6 MPa (10 bis 60 bar) erfolgt.

11. Verfahren nach einem der Ansprüche 1 bis 10,
wobei das Erwärmen des Reaktionsgemisches in Verfahrensschritt g) auf eine Temperatur in dem Bereich von 80 °C bis 160 °C erfolgt.

12. Verfahren nach einem der Ansprüche 1 bis 11,
umfassend den zusätzlichen Verfahrensschritt f'):
f') Zugabe eines Lösungsmittels.

13. Verfahren nach Anspruch 12,
wobei das Lösungsmittel ausgewählt ist aus: CH₃CN, DMSO, DMF, *^{tert}*BuOH, Toluol, Cyclohexan.

## Geänderte Patentansprüche

### Geänderte Patentansprüche gemäss Regel 137(2) EPÜ.

1. Verfahren umfassend die Verfahrensschritte:
a) Vorlegen eines Alkylhalogenids;
b) Zugabe eines Liganden gemäß Formel (I): 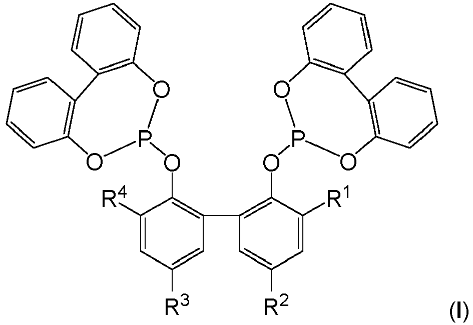 wobei
R¹, R², R³, R⁴ jeweils unabhängig voneinander ausgewählt sind aus: -H, -(C₁-C₁₂)-Alkyl, -O-(C₁-C₁₂)-Alkyl;
c) Zugabe einer Verbindung, welche Cu umfasst, die ausgewählt ist aus: CuCl₂, [Cu(OTf)], Cul, CuBr, Cu(OAc)₂;
d) Zugabe eines Alkoholats,
f) Zuführen von CO und H₂;
g) Erwärmen des Reaktionsgemisches aus a) bis f), wobei das Alkylhalogenid zu einem Ester umgesetzt wird.

2. Verfahren nach Anspruch 1,
wobei R¹ und R⁴ für -(C₁-C₁₂)-Alkyl stehen.

3. Verfahren nach einem der Ansprüche 1 oder 2,
wobei R¹ und R⁴ für -*^{ter}*Bu stehen.

4. Verfahren nach einem der Ansprüche 1 bis 3,
wobei R² und R³ für -O-(C₁-C₁₂)-Alkyl stehen.

5. Verfahren nach einem der Ansprüche 1 bis 4,
wobei R² und R³ für -OCH₃ stehen.

6. Verfahren nach einem der Ansprüche 1 bis 5, wobei der Ligand die Struktur (1) aufweist:

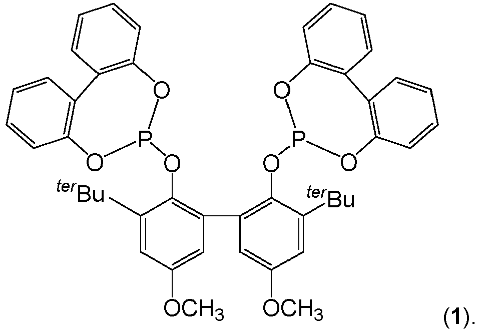

7. Verfahren nach einem der Ansprüche 1 bis 6,
wobei es sich bei dem Alkylhalogenid in Verfahrensschritt a) um ein lodid handelt.

8. Verfahren nach einem der Ansprüche 1 bis 7,
wobei es sich bei dem Alkoholat aus Verfahrensschritt d) um ein Natrium-Alkoholat oder Kalium-Alkoholat handelt.

9. Verfahren nach einem der Ansprüche 1 bis 8,
wobei das Zuführen von CO in Verfahrensschritt f) mit einem Druck in dem Bereich von 1 bis 6 MPa (10 bis 60 bar) erfolgt.

10. Verfahren nach einem der Ansprüche 1 bis 9,
wobei das Erwärmen des Reaktionsgemisches in Verfahrensschritt g) auf eine Temperatur in dem Bereich von 80 °C bis 160 °C erfolgt.

11. Verfahren nach einem der Ansprüche 1 bis 10,
umfassend den zusätzlichen Verfahrensschritt f'):
f') Zugabe eines Lösungsmittels.

12. Verfahren nach Anspruch 11,
wobei das Lösungsmittel ausgewählt ist aus: CH₃CN, DMSO, DMF, *^{tert}*BuOH, Toluol, Cyclohexan.
